# EUROPEAN PATENT SPECIFICATION

(11) **EP 0 426 504 B1**
(45) Date of publication and mention of the grant of the patent: **11.08.1993**
(21) Application number: 90400926.3
(22) Date of filing: 04.04.1990
(51) Int. Cl.: A61K 35/74, A23K 1/00

(54) **Detoxification of certain environmental protection agency declared toxicants by naturally occurring anaerobic organisms**
Entgiftung bestimmter, durch das Umweltschutzamt als toxisch erklärter Gifte, mit Hilfe natürlich vorkommender anaerober Organismen
Détoxification de certains agents déclarés comme étant toxiques par l'agence de protection de l'environnement, par des organismes anaerobiques naturels

(30) Priority: 24.10.1989 US 426436
(43) Date of publication of application: 08.05.1991
(73) Proprietor: STATE OF OREGON ACTING BY AND THROUGH THE OREGON STATE BOARD OF HIGHER EDUCATION ON BEHALF OF OREGON STATE UNIVERSITY, EUGENE, Oregon 97403 (US)
(72) Inventor: Craig, Albert Morrie, Corvallis, Oregon 97330 (US)
(74) Representative: Kedinger, Jean-Paul

(56) References cited:
- Biological Abstracts, vol. 83, no. 8, 1987, abstract no. 81688, Philadelphia, PA, US; A.M. Craig et al.
- Biological Abstracts, vol. 63, no. 5, 1977, abstract no. 24381, Philadelphia, PA, US; G.C. Lanigan et al.

## Description

### BACKGROUND OF THE INVENTION

One of the more important problems currently facing mankind is pollution caused from by-products of our modern society. In fact, the Environmental Protection Agency has listed many organic chemical by-products as being on the EPA priority pollutants list. Among those priority pollutants are many organic compounds such as certain chlorinated hydrocarbons, certain phthalate esters, certain polynuclear aromatic hydrocarbons, certain triazines, and many commonly occurring phenols. These contaminants when present in soil and water are difficult to remove and cause many potential risks such as carcinogenic properties, risk to human organs such as the liver (necrosis), and risk to domestic animals that inadvertently ingest sufficient quantities of the pollutants to interfere with their normal metabolic processes. In short the potential risks are not only to human health but also to the health of some of man's food sources.

One potential solution for environmental contamination may be the breakdown of toxic compounds by microorganisms. Just now microbial ecologists and microbiologists are beginning to unearth an array of microorganisms with certain unexpected abilities to biodegrade some of the toughest and most recalcitrant environmental chemicals. One such group of very hazardous environmental chemicals includes the pyrrolizidine alkaloids, which all commonly contain the alkaloid ring which has the following structure:

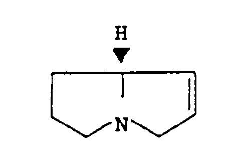

Pyrrolizidine alkaloids (PA) are known to be produced by some plants which grow in pasture lands used for domestic animals, particularly cattle and sheep. One of these commonly occurring plants is Tansy Ragwort (Senecio Jacobaea). Tansy Ragwort in particular when concentrated in the food supply of grazing cattle can result in an accumulation of pyrrolizidine alkaloids within the organs of the animal, causing liver damage. Over the life of the animal it may decrease it's growth and overall efficiency.

It has in the past been reported that toxic plants account for approximately 9.7% of all livestock deaths in the United States. Forages containing plants with pyrrolizidine alkaloids are common and are distributed throughout this country, as well as the rest of the world. Cattle, horses and humans are highly susceptible to the toxic principle with chronic terminal hepatic disease produced after consuming as little as 5% of their body weight in plant material. On the other hand, it has been observed that sheep can eat quantities of many of these toxic plants, and in particular Tansy Ragwort up to as much as 300% of their body weight with no measurable abnormalities. This unusual quality of sheep in comparison with cattle and horses has led to an investigation of the reasons why sheep have this apparent resistance.

Surprisingly it has been discovered that sheep rumen fluid contains certain microbial factors which are capable of detoxifying certain EPA priority pollutants, including pyrrolizidine alkaloids.

These initial observations of the nature and character of rumen microbial factors of sheep have led to investigatory research into the use of similar anaerobic bacterial factors for reducing pyrrolizidine alkaloid levels in silage contaminated with toxic plants, and have led to use of compositions containing these naturally occurring bacteria of sheep's rumen fluid for use as general EPA priority pollutant detoxifiers.

It can therefore be seen that there is the distinct possibility of genetically engineering the protective factors of sheep's rumen fluid and adding these to silage inoculants, or to cow's rumen, or to simply use them in compositions as general detoxifying agents for EPA priority pollutants. The net result is that pollutants can be oxidized to less harmful compounds, that contaminated forages can be protected, and that pastures can be safely fed.

Accordingly, a primary objective of the present invention is to prepare detoxifying compositions which can be useful for detoxifying priority pollutants, and in particular PA, and to prepare detoxifying compositions which can be used in silage inoculants and/or in direct inoculation into the rumen of less resistant animals such as cattle and horses.

The method of accomplishing this as well as other objectives of the present invention will be apparent from the detailed description which follows.

### BRIEF DESCRIPTION OF THE DRAWINGS

Fig. 1 is a graph showing the ability of sheep's rumen fluid to metabolize pyrrolizidine alkaloid (PA).

Fig. 2 is a graph showing ovine ruminal fluid detoxification of PA, after preconditioning of Tansy Ragwort.

### SUMMARY OF THE INVENTION

A culture media composition which contains detoxifying effective amounts of bacteria normally contained in sheep's rumen fluid. It is used as a detoxifying agent, as silage inoculant and for use in direct treatment of animals normally not resistant to toxic agents such as pyrrolizidine alkaloids.

### DETAILED DESCRIPTION OF THE INVENTION

This invention involves the recent discovery that certain anaerobic organisms biodegrade certain EPA priority Pollutants, and more specifically pyrrolizidine alkaloids. These naturally occurring anaerobic microorganisms were derived from sheep's rumen fluid. The investigation began after making the initial observation that domestic sheep seem to be unusually resistant to Tansy Ragwort poisoning whereas cattle and horses grazing on the same land were not so resistant. The cattle and horses showed classic pyrrolizidine alkaloid toxicity symptoms such as liver necrosis and hardening of the blood vessels, coupled with general over-all decreased animal function efficiency.

While sheep's rumen fluid contains many different bacteria, it has been discovered that critical to the detoxifying properties of sheep's rumen fluid are the necessity for a combination of bacteria of the following genera: Streptococcus sp., Peptococcus sp., Lactobacillus sp., Bacteroides sp., and Ruminococcus sp. and preferably but not essential Veillonella sp. and Eubacterium sp.

Compositions containing this combination of enumerated bacteria, have the capability of degrading of pyrrolizidine alkaloids. It is not certain how this biodegradation works, but it is believed that metabolites of the organisms oxidize the pyrrolizidine alkaloids by splitting the alkaloid ring. For pyrrolizidine alkaloids it is preferred that the composition contain streptococcus sp. and peptococcus and most preferrably also contains at least two additional gram positive bacteria.

Surprisingly, the combination of anaerobic microorganisms earlier specified is not only effective against pyrrolizidine alkaloids, but it is also effective against numerous EPA recognized priority pollutants. These EPA priority pollutants fall into five major categories: phenols, triazines, polynuclear aromatic hydrocarbons, phthalate esters, and chlorinated hydrocarbons. Amongst the phenols which may be biodegraded by the bacteria compositions of the present invention are the following: 2,4,6-trichlorophenol, 4-chloro-3-methylphenol, 2-chlorophenol, 2,4-dichlorophenol, 2,4-dimethylphenol, 2-nitrophenol, 4-nitrophenol, 2,4-dinitrophenol, 2-Methyl-4,6-dinitrophenol, pentachlorophenol, and phenol. Amongst the polynuclear aromatic hydrocarbons which may be biodegraded by the bacterial compositions of the present invention are the following: acenaphthene fluoranthene, naphthalene, benzo(a)anthracene, benzo(a)pyrene, benzo(b)fluoranthene, benzo(k)fluoranthene, chrysene (93%), acenapthylene, anthracene, benzo(ghi)perylene, fluorene, phenanthrene, dibenzo(a,h)anthracene, indeno(1,2,3-cd)pyrene, and pyrene. Finally, amongst the chlorinated hydrocarbons which may be biodegraded by the compositions of the present invention are the following: 1,2,4-trichlorobenzene, hexachlorobenzene, hexachloroethane, 2-chloronaphthalene, 1,2-dichlorobenzene, 1,3-dichlorobenzene, 1,4-dichlorobenzene, and hexachlorobutadiene.

For phenols it has been noted as preferred to have Ruminococcus sp. present and for Triazines additional spore forming bacteria such as Clostridia sp. However all of these are present in sufficient amounts in sheep's rumen fluid.

Use of bacterial metabolism to biodegrade these EPA pollutants offers a significant, natural means of pollution clean up. It offers the potential for many of mankind's concerns for chemical carcinogens to be eliminated, and the potential for rendering many of these pollutants less persistent and less toxic in the environment.

The detoxification of pyrrolizidine alkaloid from Tansy Ragwort in sheep's rumen fluid has led directly to the discoveries of the present invention. In short, it has been definitively shown that the biodegradation of pyrrolizidine alkaloids (PA) from the plant Tansy Ragwort can be reduced or eliminated by sheep's rumen fluid. Moreover, as demonstrated by the examples shown below this ruminal fluid has been shown to biodegrade a number of other priority pollutants.

Common to all of the sheep's rumen fluid demonstrated to exhibit this property are a combination of bacteria of the following genera: Streptococcus sp., Peptococcus sp., Lactobacillus sp., Bacteroides sp. and Ruminococcus sp. Generally each of these should be present in approximately similar organism concentrations. In particular the concentrations of each should be from 10⁸ to 10¹². In the preferred composition there may also be bacteria of the following additional genera: Veillonella sp. and Eubacterium sp. and some spore forming bacteria. These preferred organisms when present should be present in the same earlier expressed concentration levels.

The bacteria may be contained in a conventional nutrient carrier system. Preferably the system should be designed to simulate and maintain the same ecological conditions as in the sheep rumen, in particular pH within the range of from about 6.8 to about 7.2. While sheep's rumen fluid will function effectively as the medium, certain synthetic media also can be used in conjunction with the organisms earlier specified. In particular, mediums that simulate the habitat of the rumen from other aspects as well as pH can be employed. Included in the successful habitat simulating medium was RCGA (rumen fluid, cellobiose, glucose, and auger, Bryant and Burkey, 1953) and 98.5 (a modification of RCGA, Bryant and Robinson, 1961). Both of these media contain 30% of sterilized ruminal fluid and are similar in concentrations of salt and minerals; however, the 98-5 medium contains less sugars. Another useful medium is Pyrrolizidine alkaloid enrichment media modified from one reported by Shelton and Tiedje, 1984, which contains a basal medium of salts, trace minerals and vitamins to which rumen fluid (5% of supernatants) is added. So far it appears that the detoxifying bacteria are able to survive in a variety of mediums which function effectively as nutrient-carriers, with the most important criteria being the successful pH control within the range earlier specified. One particular media designed to simulate the composition of sheep's saliva is McDougall's buffer composed of the following in grams per litre: NaHCO₃ - 9.8; KCl - 0.57; Na₂HPO₄:12 H₂O - 9.3, NaCl - 0.47; MgCl₂ (anhyd) - 0.06; CaCl₂ (anhyd) - 0.04. In addition, (NH₄)₂ SO₄ at 2.64 g/l was added to the solution).

The following examples are offered to illustrate but not limit the process of compositions of the present invention.

### EXAMPLE 1

A series of in-vitro experiments were conducted comparing the degradation capabilities and rates of ovine versus bovine ruminal fluid utilizing a modified Hungates artificial rumen technique with serum bottles.

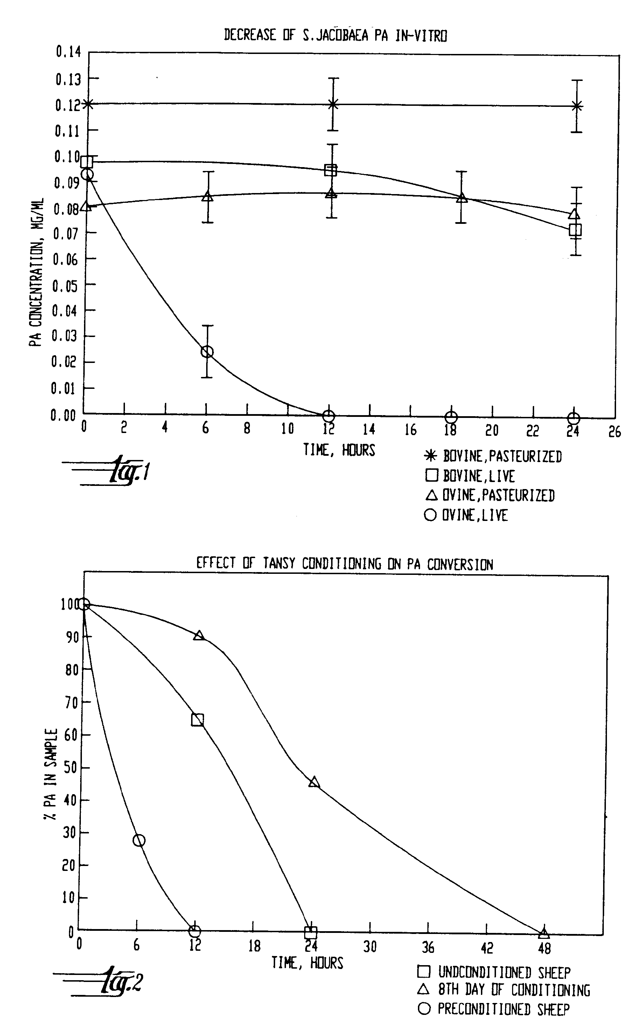

In these experiments ruminal fluid was removed manually through a permanent rumen fistula in sheep and cattle. The ruminal fluid was immediately filtered through nylon mesh into a prewarm (38° C) thermos bottle for transport back to the laboratory. The rumen fluid was cut 50/50 with pre-warmed and carbon dioxide flushed McDougall's buffer and crystalline pyrrolizidine alkaloid was added to the solution at a concentration of 10 milligrams per ml (pyrrolizidine alkaloid in phosphate buffer at pH of 6.8). One aliquot of ruminal fluid was pasteurized in each trial prior to incubation. All bottles were flushed with carbon dioxide and incubated at 38° C in a shaking incubator. Samples were taken at 0 time, 12, 24 and 48 hours and the pyrrolizidine alkaloid extracted and quantitated using high performance liquid chromatography (HPLC). Fig. 1 shows the results.

In summarizing, as can be seen from Fig. 1 utilizing a number of sheep (n = 15) and cattle (10) trials, it was evident that the sheep ruminal fluid contained microorganisms that metabolized the pyrrolizidine alkaloid in 12 to 24 hours while only a minor portion of the pyrrolizidine alkaloid was degraded by the bovine microorganism. Pasteurization eliminated the microorganism population with the resultant persistence of the pyrrolizidine alkaloid concentrations.

In every instance the sheep's rumen fluid was examined and found to contain the following genera of bacteria: Streptococcus sp., Peptococcus sp., Lactobacillus sp. and Bacteroides sp. In some instances they also contained Veillonella sp. and Eubacterium sp.

### EXAMPLE 2

It has been found that the microorganisms in the sheep's rumen can be environmentally induced with a resultant increase in the rate of detoxification of pyrrolizidine alkaloid. This was accomplished by feeding the previously unexposed sheep 5% Tansy Ragwort plant in their daily diet. In these trials, the time of detoxification was decreased from the mean of approximately 48 hours to between 12 and 24 hours, depending on the particular animal evaluated. This is illustrated in Fig. 2 showing the effect of Tansy Ragwort conditioning on PA conversion.

Additional in vitro tests have induced the bacteria to further decrease the detoxification time. In particular re-spiking of in vitro rumen fluid daily for four days with 10 micrograms per milliter of pyrrolizidine alkaloids decreased the mean of 48 hours to within the range of 6 to 10 hours. This indicates that the bacteria and/or enzymatic pathway that metabolizes the alkaloid can be induced over several days.

### EXAMPLE 3

A third set of experiments were designed to isolate the type of microorganisms in the sheep's ruminal fluid that were responsible for the pyrrolizidine alkaloid detoxification. Differential centrifugation of full ruminal fluid was done and the resultant supernatants tested for their ability to metabolize pyrrolizidine alkaloids compared to their respective uncentrifugated control fluid. The rates of detoxification from 15 separate trials were analyzed. Analysis of the data combined with microscopic evaluation of the supernatants determined that the prime degrading microorganisms were a consortium of bacteria of the following genera: Streptococcus sp., Peptococcus sp., Lactobacillus sp., Bacteroides sp., Ruminococcus sp..

Gas chromatography-mass spectro photometer data on spiked whole ruminal fluid as well as the supernatants of centrifugation indicated that the pyrrole ring that is the backbone of the pyrrolizidine alkaloids was broken down during the degradation process.

If similar experiments are conducted on other EPA priority pollutants, particularly phenols and triazines and perhaps polynuclear aromatic hydrocarbons, phthalate esters and chlorinated hydrocarbons similar results may be obtained. In particular for phenols and triazines in each instance similar decreases in the level of toxicants in use of whole sheep rumen fluid were noted. Namely GC/MS analysis of rumen fluid reveals lack of structure of ring compounds characteristic of toxicant peaks.

The ability of this consortium of bacteria to degrade certain EPA priority pollutants offers the opportunity for many uses. In particular the bacteria consortium in a conventional nutrient-carrier medium can itself be used as a detoxifying composition. Alternatively it may be used as a silage inoculant. It also can be combined with rumen fluid of other animals, generally at a range of about 50/50 and then used to treat for example cattle and horses to provide them the same unique toxic resistance that sheep rumen fluids naturally provides. If used as a probiotic, the composition may contain 70% sheep's rumen fluid and 30% cow's rumen fluid for use with bovine. Ruminal fluid levels of from 50:50 sheep:cow may range from 50:50 to 70:30. It may also be possible to simply add the isolated organisms directly to the bovine rumen and achieve similar results.

It can be seen that the invention accomplishes at least all of it's stated objectives.

## Claims

1. A bacterial composition for detoxification of certain EPA priority toxicants selected from the group consisting of phenols, triazines, polynuclear aromatic hydrocarbons and chlorinated hydrocarbons, comprising a cultural media nutrient-carrier composition,
said composition containing a detoxifying effective amount of a bacterial consortium normally contained in sheep's rumen fluid including at least the following genera of bacteria: Streptococcus sp., Peptococcus sp., Lactobarillus sp., Bacteroides sp., and Ruminococcus sp.

2. The composition of claim 1 wherein composition additionally contains Veillonella sp. and Eubacterium sp.

3. The composition of claim 1 which includes spore forming bacteria.

4. The composition of claim 2 wherein the culture media is at a pH within the range of 6.8 to 7.2.

5. The composition of claim 4 wherein the culture media simulates nutrient composition of sheep's rumen fluid.

6. The composition of claim 5 wherein the culture media is McDougall's buffer.

7. A silage inoculant composition containing a naturally occurring consortium of bacteria capable of detoxifying pyrrolizidine alkaloids (PA) that naturally occur in certain pasture plants comprising :
a culture media nutrient-carrier composition containing a detoxifying effective amount of a consortium of bacteria normally contained in sheep's rumen fluid, including Streptococcus sp., Peptococcus sp., Lactobacillus sp., Bacteroides sp., and Ruminococcus sp.

8. A method for detoxification of EPA priority toxicants, particularly those selected from the group consisting of phenols, triazines, polynuclear aromatic hydrocarbons and chlorinated hydrocarbons, comprising the degradation of said toxicants with an effective amount of a consortium of bacteria derived from sheep's rumen fluid, said consortium of bacteria being used in the form of a composition as defined in any one of claims 1 to 6, with the proviso that said method is not practised on the human or animal body.

## Patentansprüche

1. Eine bakterielle Zusammensetzung zur Entgiftung bestimmter, nach dem Umweltschutzamt (EPA) vorrangiger Gifte, ausgewählt aus der Gruppe von Phenolen, Triazinen, mehrkernigen aromatischen Kohlenwasserstoffen und chlorierten Kohlenwasserstoffen, mit einer Nährmedium und Nahrungsträger bildenden Zusammensetzung, die einen entgiftend wirkenden Anteil eines bakteriellen Konsortiums beinhaltet, das normalerweise in Schafmagensaft enthalten ist und wenigstens die folgende Bakteriengattungen aufweist: Streptococcus sp., Peptococcus sp., Lactobacillus sp., Bacteroides sp., und Ruminococcus sp.

2. Die Zusammensetzung nach Anspruch 1, wobei die Zusammensetzung zusätzlich Veillonella sp. und Bubacterium sp. enthält.

3. Die Zusammensetzung nach Anspruch 1, die sporenbildende Bakterien enthält.

4. Die Zusammensetzung nach Anspruch 2, wobei das Nährmedium einen pH-Bereich von 6,8 bis 7,2 aufweist.

5. Die Zusammensetzung nach Anspruch 4, wobei das Nährmedium Nahrungszusammensetzung von Schafmagensaft nachahmt.

6. Die Zusammensetzung nach Anspruch 5, wobei das Nährmedium McDougalls Puffer ist.

7. Eine Impfzusammensetzung für Silage mit einem natürlich vorkommenden Konsortium von Bakterien für das Entgiften von Pyrrolizidinalkaloiden (PA), die natürlicherweise in bestimmten Weidepflanzen vorkommen, enthaltend:
eine Nährmedium und Nahrungsträger bildende Zusammensetzung, die einen entgiftend wirkenden Anteil eines Konsortiums von Bakterien beinhaltet, das normalerweise in Schafmagensaft enthalten ist, einschließlich Streptococcus sp., Peptococcus sp., Lactobacillus sp., Bacteroides sp., und Ruminococcus sp.

8. Ein Verfahren zur Entgiftung bestimmter, nach dem Umweltschutzamt vorrangiger Gifte, insbesondere jene ausgewählt aus der Gruppe von Phenolen, Triazinen, mehrkernigen aromatischen Kohlenwasserstoffen und chlorierten Kohlenwasserstoffen, umfassend den Abbau der genannten Gifte mit einem wirksamen Anteil eines Konsortiums von Bakterien, erhalten aus Schafmagensaft, wobei das Konsortium von Bakterien in Form einer Zusammensetzung nach einem der Ansprüche 1 bis 6 verwendet wird, mit der Maßgabe, daß das genannte Verfahren nicht im menschlichen oder tierischen Körper praktiziert wird.

## Revendications

1. Composition bactérienne pour la détoxication de certaines substances toxiques prioritaires pour l'Agence de Protection de l'Environnement, choisies dans le groupe constitué par les phénols, les triazines, les hydrocarbures aromatiques polynucléaires et les hydrocarbures chlorés, comprenant une composition d'un support et d'une substance nutritive formant milieu de culture,
ladite composition contenant une quantité .efficace pour détoxiquer d'un ensemble de bactéries normalement contenues dans le fluide de la panse de mouton, comprenant au moins les genres de bactéries suivants : espèce Streptococcus, espèce Peptococcus, espèce Lactobacillus, espèce Bacteroides, et espèce Ruminococcus.

2. Composition selon la revendication 1, dans laquelle la composition contient en outre l'espèce Veillonella et l'espèce Eubacterium.

3. Composition selon la revendication 1, contenant des bactéries sporogènes.

4. Composition selon la revendication 2, dans laquelle le milieu de culture est à un pH qui se situe dans la plage de 6,8 à 7,2.

5. Composition selon la revendication 4, dans laquelle le milieu de culture simule une composition nutritive du fluide de la panse de mouton.

6. Composition selon la revendication 5, dans laquelle le milieu de culture est le tampon de McDougall.

7. Composition d'inoculation d'un ensilage, contenant un ensemble de bactéries d'origine naturelle capables de détoxiquer les alcaloïdes de type pyrrolizidine (PA) naturellement présents dans certaines plantes de pâture, comprenant :
une composition d'un support et d'une substance nutritive formant milieu de culture contenant une quantité efficace pour détoxiquer d'un ensemble de bactéries normalement contenues dans le fluide de la panse de mouton, comprenant l'espèce Streptococcus, l'espèce Peptococcus, l'espèce Lactobacillus, l'espèce Bacteroides, et l'espèce Ruminococcus.

8. Méthode de détoxication de substances toxiques prioritaires pour l'Agence de Protection de l'Environnement, en particulier celles choisies dans le groupe constitué par les phénols, les triazines, les hydrocarbures aromatiques polynucléaires et les hydrocarbures chlorés, comportant la dégradation desdites substances toxiques par une quantité efficace d'un ensemble de bactéries issues du fluide de la panse de mouton, ledit ensemble de bactéries étant utilisé sous la forme d'une composition telle que définie dans l'une quelconque des revendications 1 à 6, sous réserve que ladite méthode ne soit pas mise en oeuvre sur le corps humain ou animal.
